# EUROPEAN PATENT APPLICATION

(11) **EP 2 383 571 A1**
(43) Date of publication of application: **02.11.2011**
(21) Application number: 10004610.1
(22) Date of filing: 02.05.2010
(51) Int. Cl.: G01N 33/50, G01N 33/564, G01N 33/574, G01N 33/68, G01N 33/74

(54) **Growth differntioation factor 15 (GDF 15) for risk prediction of diabetic foot ulcer**

(71) Applicant: Prof. Hess Medical Consulting GmbH, 55270 Sörgenloch (DE)
(72) Inventor: Hess, Georg, Prof. dr., 55130 Mainz (DE); Horsch, Andrea, 68259 Mannheim (DE); Zdunek, Dietmar, 82327 Tutzing (DE)

(57) **Abstract**

The present invention relates to a method of predicting if a diabetes mellitus patient preferably with type 2 and more preferably with type 1 diabetes will suffer from diabetic foot ulcer, the method comprising
a) determining the amount of GDF 15 in a sample of a type 1 diabetes mellitus patient and
b) comparing the amount or GDF 15 determined in step a) to a reference amount and establishing a prediction

Also encompassed by the present invention arc devices and kits for carrying out the aforementioned method.

## Description

The present invention relates to a method for the prediction or assessing the risk of a patient with diabetes mellitus preferably type 2 diabetes mellitus and more preferably type 1 diabetes mellitus to proceed preferably from foot injury to diabetic foot ulcer The method is based on the determination of growth differentiation factor 15 and optionally a natriuretic peptide or variant thereof and optionally forming a ratio of GDF 15 and the natriuretic peptide or a variant thereof in a sample of a subject suffering from diabetes mellitus Also encompassed the present invention arc devices and kits for carrying out the aforementioned methods.

According to the WHO the total number of people with diabetes mellitus was 171 million in the year 2000. This number is projected to rise up to 366 million in 2030. According to the most recent WHO recommendations for the diagnosis of diabetes mellitus, diabetes is defined by fasting plasma glucose over 7.0 mmol/l (126 mg/dl) or 2 h plasma glucose over 11.1 mmol/l (200 mg/dl) (Alberti K.G., Diabet. Med. 1998, 15: 539 53; The Expert Committee on the diagnosis and classification of Diabetes Mellitus. Follow up Report on the Diagnosis of Diabetes mellitus. Diabetes Care 2003: 26: 3160-7).

There are to main categories of diabetes mellitus type 1 and type 2, which can be distinguished by a combination of features known to the person skilled in the art.

In type 1 diabetes (previously called juvenile-onset or insulin dependent), insulin production is decreased or absent because of suspected autoimmune pancreatic beta cell destruction possibly triggered by environmental exposure in genetically susceptible individuals. Destruction proceeds subclinically over months or years until beta cell mass decreases to the point that insulin concentrations are no longer adequate to control glucose levels. The type 1 diabetes generally develops in childhood or adolescence und until recently was the most common form diagnosed before age 30; however it can also develop in adults (late onset type 1 diabetes).

In type 2 diabetes (previously called adult-onset on nun insulin dependent) insulin secretion is inadequate Often insulin levels are very high, especially early in the disease, but peripheral insulin resistance and increased hepatic production of glucose makes insulin levels inadequate to normalize plasma glucose levels. Insulin production then falls, further exacerbating hyperglycemia. The disease generally develops in adults and becomes more common with age, plasma levels reach higher levels after meals, especially after meals containing high carbohydrate loads and take longer to return to normal or lower levels, in part because of accumulation of visceral and abdominal fat and decreased muscle mass.

Further details related to the definition, pathophysiology of diabetes mellitus and the discrimination between type 1 and type 2 diabetes mellitus can be obtained from A.C. Powers. Diabetes mellitus in Harrison. Principles of lnternal Medicine, 17 Ed.,Mc Graw Hill Medical, Eds. A.S. Fauci et al) and is referred to

Diabetes mellitus can be followed by a variety of serious complications, one such complication is diabetic nephropathy, early sign of nephropathy is albuminuria which can be dissected into mirco- and macroalbuminuria. Diabetic nephropathy may be followed by progressive deterioration of kidney function. In fact the majority of patients on dialysis suffer from diabetes mellitus.

Diabetic retinopathy is another serious computation in the course of diabetes mellitus, retinopathy is characterized by scattered hemorrhages, yellow exsudates and neovascularisation. Diabetic retinopathy may proceed to blindness.

Vascular complications associated with hyperglycemia and hyperlipemia represent another frequent complication of diabetes mellitus. Patients with diabetes mellitus have in increased frequency of acute coronary syndrome. myocardial infarction and heart failure when compared to age matched individuals without diabetes mellitus. Long standing diabetes mellitus is also followed by loss of myelinated and unmyelinated fibers of nerves which results in polyneuropathy, mononeuropathy and/ or autonomic neuropathy. In the gut neuropathy results in gastrointestinal dysfunction, similar effects may occur in the genitourinary tract In case of peripheral neuropathy the most frequent expression of neuropathy is loss of distal sensory function. (A.C. Powers et al)

Diabetic foot ulcer occurs in 5 % of patients with diabetes mellitus each year and this is nine to eleven times more likely than in patients without diabetes mellitus, Foot amputations occur in patients with diabetes 24 times more often than in age matched nondiabetic individuals ( Cheer K. et al, BMJ 2009,339: 1304 1307,Miller A., Med Rehabil Clin N. Am 2009, 20, 611 ― 625). Consequences of foot ulcer arc foot infections, osteomyelitis and amputation, specifically if treatment of osteomyelitis is ineffective (Cheer et al ). Foot ulcer develops preferably from foot injury, injury is promoted by neuropathy ( loss of sensitation ), foot deformity ( such as the Charcot foot, a foot abnormality typical for diabetes mellitus) and may he the result of inappropriate foot care. Development of foot injury into foot ulcer is finally promoted by peripheral vascular disease and abnormal wound healing including infections (Cheer et al).

Current recommendations include equation of peripheral neuropathy using a 128 Hz tuning fork to check vibration, inspection of the foot for deformities associated with appropriate recommendations ( Cheer et al). Peripheral vascular disease can be assessed by clinical examination such as palpating peripheral pulses, estimating skin temperature and potentially followed by imaging techniques such as Doppler ultrasound imaging. Although several mechanisms of diabetes specific detects in innate and adaptive immunity have been identified, they have not been proven useful in the clinical setting (Peleg A.Y. et al Diabetes Metab Res Rev 2007: 23: 3 ―13).

Thus, there is an urgent need to identify reliable diagnostic tests that would predict the risk to develop foot ulcer from preferably foot injury beyond current risk factors. Such a test would also be suitable to modify recommendations as to the frequency of foot inspection, advices for foot care and physician visits. It addition such a marker would determine type, onset and duration of treatment and time and extent for intervention and its nature. Doing so such a test would meet needs currently not covered by current techniques und would allow personalized risk prediction, advice and therapy

GDF 15 was first identified as macrophage inhibitory cytokine- 1 (MIC 1) and later also named placental transforming growth factor (Bootcov 1997, Proc Natl Acad Sci 94: 11514 11519, Tan et al 2000, Proc Natl Acad Sci 97: 109 - 114). Recently it has been shown that GDF 15 is an indicator of cardiovascular complications such as in acute coronary syndrome and in heart failure ( US 2003/0232385; Kempf et al 2006, Cire Res 98, 351 - 360). The above studies indicate that GDF 15 values are closely linked to NT-pro BNP concentrations, the natriuretic peptides NT-pro BNP is believed to represent cardiac function, however its predictive power is independent from that of GDF15. Very recently GDF 15 had been linked to cardiovascular disease and disease in the elderly ( Lind et al Eurp. Heart J. 2009. 30 2346-2353 ). Recently MIC 1 (GDF 15) has been found to be increased in obese patients and those with type 2 diabetes, however the cardiac function was not assessed, leaving the association of GDF 15 to cardiovascular disease open (Dostalova et al Eurp. J. of Endocrinology 2009: 161 397 404). Moreover GDF 15 was shown to predict all cause mortality, fatal and nonfatal cardiovascular events as well as end stage renal failure in a group of type 1 diabetes patients followed for a period of 12 years (EP 2009/056090).

The technical problem underlying the present invention can be seen as a provision of means and methods for complying with the aforementioned needs. The technical problem is solved by the embodiments characterised in the claims and herein below.

Accordingly the present invention relates to a method of predicting the risk of a diabetes mellitus patient, preferably with type 2 and more preferably with type 1 diabetes mellitus to suffer from diabetic foot ulcer, the method comprising:
a) determining the amount of GDF 15 in a sample of a diabetes patient and
b) comparing the amount of GDF 15 determined in step a) to a reference amount, thereby predicting said risk.

The method of the present invention, preferably is an in vitro method. Moreover, it may comprise steps in addition to those explicitly mentioned above For example, further steps may relate to sample pre-treatments or evaluation of the results obtained by the method. The method of the present invention may also be used for monitoring confirmation, and subclassification of a diabetes patient in respect to said complication. The method may be carried out manually or assisted by automation. Preferably, step (a) and/or (b) may in total or in part be assisted by automation. e.g. by a suitable robotic and sensory equipment for the determination of step (a) or a computer-implemented comparison in step (b).

The term "predicting" used herein refers to assessing the probability according to which patients with diabetes mellitus will suffer a diabetic foot ulcer complication within a defined window time (predictive window) in the future The predictive window is an interval in which the subject will develop said complication to the predicted probability. The predictive window may be the entire remaining life span of the subject upon analysis by the method of the present invention. Preferably, however, the predictive interval is an interval of one month, six months or one, two, three, four, five or ten years after appearance of the complication ( more preferably and precisely, after the sample to be analysed by the method of the present invention has been obtained). Preferably such foot ulcer complication will in average develop in 1 %, 2 %, 3 %, 4 %, 5 %, 7 % and 10 % per year, most preferably in 5 % per year in a patient with diabetes mellitus. The term "predicting an increased risk" to develops a fool ulcer in a patients with diabetes mellitus is understood in the context of the present invention that the risk of development of a foot ulcer in a diabetes mellitus patient is higher than average, vice versa the term "predicting a decreased risk" indicates that this risk is below average. Prediction the risk can be applied to different time intervals, e.g. 3 months, 6 months, 1 year. 2 years, 3 years, 5 years, 10 years, 20 years or longer, preferably to 5 years, more preferably to 3 years most preferable to one year. As will understood by those skilled in the art, such an assessment is usually not intended to be correct for 100 % of the subjects to be analysed The term, however, requires that the assessment will be valid for a statistically significant portion of the subjects to he analysed. Whether a portion is statistically significant can be deter mined without further ado by the person skilled in the art using various well known statistic evaluation tools, e.g. determination of confidence intervals, p-value determination, Student's t-test. Mann-Whitney test, etc. Details arc found in Dowdy and Weardan, Statistics for Research, John Wiley & Sons, New York, 1983. Preferred confidence intervals are at least 90 %, at least 95 % at least 97 %, at least 98 %, or at least 99 %. The p values are preferably 0.1, 0.05, 0.01, 0.005, or 0.0001. Preferably the probability envisaged by the present invention allows that the prediction will be correct for at least 60%, at least 70 %, at least 80 %, or at least 90 % of the subjects of a given cohort.

The term "patient" or "subject" or "individual" as used herein relates to animals, preferably mammals and, more preferably humans.

It is envisaged that in accordance with the aforementioned method of the present invention that the subject shall suffer from diabetes mellitus preferably tyre 2 diabetes mellitus and more preferably type I diabetes mellitus. Preferably the patients shall not suffer from foot injury and more preferably from foot ulcer when first tested and the risk is determined. Furthermore the subject shall be preferably clinically stable,preferably and specifically not suffer from acute metabolic changes such as diabetic ketoacidosis. Furthermore the subject shall preferably not show signs of acute infection as for e.g. evidenced by chills, fever and other signs known to the person skilled in the art. More details can be found e.g. under www.merck.com/mmpe//index.html or Harrison Principles of Internal Medicine. The subject may however show signs of nephropathy as documented by albumin excretion in urine exceeding 30 mg/24 h, however shall preferably have unimpaired kidney function as documented by creatinine levels within the normal range of the creatinine test of the respective manufacturer. The diagnosis of normal kidney function is known to the person skilled in the art.

Years of preferably poorly controlled diabetes mellitus lead to multiple complications such as diabetic nephropathy, macro- and microvascular disease, diabetic retinopathy, diabetic neuropathy. Examples for micro- and macrovascular complications include acute coronary syndromes, myocardial infarction, heart failure and peripheral artery disease. It should however be noted that not all complications occur in a single patients suggesting that different pathological mechanism are responsible for different chronic complications in patients with diabetes mellitus (A.C. Powers et al). In contrast to acute diabetes mellitus complication chronic complications develop with time preferably within one month, 2 months, 3 months, 6 months, 1 year, 2 years, 5 years and more preferably 10, years , 15 years, 20 years and longer after the disease has developed, this is know to the person skilled in the art

Diabetic foot ulcer in the context in the context of the present invention is preferably understood as an injury of the skin of the foot in a patient with diabetes mellitus which proceeds to soft tissue (primarily sterile) infection preferably associated with necrosis and possibly gangrene where other causes other than diabetes mellitus have been excluded. Ulcer in the context of the present invention is understood preferably as a local defect of the skin preferably associated with an excavation of variable size. Diabetic foot ulcer may occur with or without superinfection. Superinfection may be caused by fungal infections and preferably by bacteria.

Diabetic foot ulcer may vary in terms of time to heal or even become may not heal at all without surgery (Jeffcotte W.J. et al Diabetic Medicine 2008 25: 1380 - 1389). Infections related to foot ulcer may extent to the bone and this is the cause of ostcomyclitis (which is an infection of the bone), osteomyelitis is difficult to treat with antibiotics and osteomyelitis is a frequent cause of amputation, amputation may involve the tow(s), the forefoot or parts of the leg.

Diagnosis of foot ulcer is made by inspection of the foot so is done the diagnosis of foot injury with may proceed to foot ulcer. Factors contributing to development of foot ulcer can be identified by palpation of foot pulses and if absent Doppler imagine techniques can be applied. Diagnosis of vascular diabetes complications can also be supported by angiography where contrast agents are used to image peripheral arteries using x-ray techniques. Peripheral neuropathy can be diagnosed using a 128 herz tuning fork. These and other methods are known to the person skilled in the art (Jeffkote W.J. et at Diabetic Medicine 2008, 25, 1380 1389,katish J. Vase Surg 2010, 51: 476 ― 486).

The term "sample" refers to a sample of a body fluid, to a sample or separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by e.g. biopsy. Separated cells may be obtained from the body fluids or the tissues or organs by separating techniques such as centrifugation or cell sorting. Preferably cell-tissue or organ samples are obtained from those cells, tissues or organs which express or produce the peptides referred to herein.

The term "Growth Differentiation Factor 15" or "GDF 15 " related to a polypeptide being a member of the transforming growth factor (TGF) beta cytokine superfamily. The terms polypeptide, peptide and protein arc used interchangeably throughout this specification. GDF 15 was originally cloned as macrophage inhibitory cytokine-1 (MIC-1) and later also identifies as placental transforming growth factor-B, placental bone morphogenic protein, non steroidal anti-inflammatory drug-induced gene-1 and prostrate-derived factor (Bootov loc cit, Hromas, 1997 Biochem Biophy. Acta 1354:40 ― 44, Lawton 1997, Gene 203: 17 26, Yokoharma-Kobayashi 1997, J. Biochem (Tokyo) 122: 622 626, Paralkar 1998. J. Biol Chem 273: 13760 13767). Similar to other TGF-ß-realted cytokines, GDF 15 is synthesized as an inactive precursor protein, which undergoes disulfide-linked homodimerization. Upron porteolytic cleavage of the N-terminal pro-polypeotide, GDF 15 is secreted as a 28 kD dimeric protein (Raaskin 200, Embo J 19 2212 - 2220) Amino acid sequences for GDF 15 are disclosed in WO 99/06445. WO 00/70051, WO 2005/113585, (Bottner 1999, Gene 237; 105 - 111, Bootcov loc.cit, Tan loc cit. Back 2001, Mol Pharmacol 59: 901 ― 908, Hromas loc cit, Paralcar loc cit Morrish 1996, Placenta 17: 431- 441 or Yokuharna-Kohayashi loc cit. GDF 15 as used herein encompasses also variants of essential biological and immunological properties of the specific GDF 15 polypeptides. In Particular they share the same essential biological und immunological properties if they are detectable by the some specific assays referred to in this specification, e.g. he ELISA assays using polyclonal or monoclonal antibodies specifically recognizing the said GDF 15 polypeptides. A preferred assay is described in the accompanying examples. Moreover, it is to be under stood that a variant as referred to in accordance with the present invention shall have an amino acid sequence which differs due to at least one aminoacid substitution, deletion and/or addition wherein the amino acid sequence of the variant is still, preferably, at least 50 %, 60 %, 70 %, 80 %, 85 %, 90 %, 92 %, 95 %, 97 %, or 99 % identical with the amino acid sequence of the specific GDF 15 polypeptide. The degree of identity between two amino acid sequences can be determined by algorithms well known in the art. Preferably, the degree of identity is to be determined by comparing two optimally aligned sequences over a comparison window, when the fragment of amino acid sequence in the comparison window may comprise additions or deletions (e.g. gaps or overhangs) as compared to the reference sequence (which does not comprise additions or deletions) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2: 482 (1981), by the homology algorithm of Needleman and Wunsch J. Mol Biol 48:443 (1970), by the search for similarity method of Pearson and Lipman Proc Natl Acad Sci (USA) 85: 2444 (1988), by computerized implementations of these algorithms ( GAP. BESTIFT, BLASI, PASTA and TFASTA in the Wisconsin Genetics Software package, Genetics Computer Group (GCG), 575 Science Dr, Madison Wi ), or by visual inspection Given that two sequences have been identified fur comparison, GAP and BESTIFT arc preferably employed to determine their optimal alignment and, thus, the degree of identity Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF 15 polypeptides or the aforementioned types of variants as long as these fragments have the essential immunological and biological properties as referred to above. Such fragments may be e.g. degradation products of the GDF 15 polypeptide. Further included are variants which differ due to posttranslational modifications such as phosphorylation or myristylation.

In another preferred embodiment of the present invention natriuretic peptides are determined in addition to GDF 15. Preferred natriuretic peptides are A type natriuretic peptides, more preferably B type natriuretic peptides and most preferably NT-pro BNP.

The term "natriuretic peptide" comprises Atrial Natriuretic peptide (ANP) type and Brain Natriuretic peptide /BNP) type peptides or variants thereof havinf the sam predictive potential. Natriuretic peptides according to the present invention comprise ANP type and BNP-type peptides or variant thereof (see Bonov, 1996. Circulation 93: 1946 1950). ANP-type petides comprise pre-pro ANP, pro ANP, NI-pro ANP and ANP. BNP type peptides comprise pre-pro BNP, pro BNP, NT-pro BNP, and BNP. The pre-pro peptide (134 amino acids in the case of pre-pro BNP) comprises a short signal peptide, which is enzymatically cleaved off to release the pro peptide (109 amino acids in the case of pro BNP) and an active hormone (32 amino acids in the case of BNP, 28 amino acid in the case of ANP). ANP and BNP have a vasodilatory effect and cause excretion of water and sodium via the urinary tract. Preferably, natriuretic peptides according to the present invention are NT-pro ANP, ANP, and more preferably NT-pro ANP and NT-pro BNP and variants thereof. ANP and BNP arc active hormone and have a shorter half life than their respective inactive counterparts, NT-pro ANP and N1-pro BNP, RNP is metabolized in the blood, whereas NT-pro BNP circulates in the blod as an intact molecule and as such is eliminated renally. The in vivo half life of NT-pro BNP is 120 min longer than that of BNP, which is 20 min (Smith 2000 J Endocrinol.167: 239 46). Preanalytics are more robust with NT-pro BNP allowing easy transportation of the sample to a central laboratory (Mueller 2004, Clin Chem Lab Med 42: 9A2 - 4). Blood samples can be stored at room temperature for several days or may be mailed or shipped without recovery loss. In contrast storage of BNP for 48 hours at room temperature or at 4 degrees Celsius leads to a concentration loss of at least 20 % (Mueller loc.Cit) Wu 2004. Clin Chem 50: 867 73). Therefore, depending on the time course or properties of interest, either measurement of the active form or the inactive form of the natriuretic peptide can be advantageous.

The most preferred natriuretic peptides according to the preset invention arc NT-pro BNP or variants thereof. As briefly discussed above the human NT-pro BNP. as referred to in accordance with the present invention is a polypeptide comprising, preferably, 76 amino acids in length corresponding to the N terminal portion of the human NT-pro BNP molecule. The structure of the human BNP and NT-pro BNP has been described already in detail in prior art, e.g. WO 02/089657, WO 02/083913 or Bonow loc. Cit. Preferably, human NT-pro BNP as used herein is human NT-pro RNP, as disclosed in EPm0 648 228 R1.

These prior art documents are herewith incorporated by reference with respect to the specific sequences of NT-pro BNP and variants thereof disclosed herein. The NT-pro BNP referred to in accordance with the present invention further encompasses allelic and other variants of said specific sequences for human NT-pro BNP discussed above. Specifically envisaged are variant polypetides which areone the amino acid level preferably, at least 50%, 60%, 70%, 80%, 90%, 93%, 95%, 97%, 98% or 99% identical to human NT-pro BNP, preferably over the entire length of human NT-pro BNP. The degree of identity between to amino acid sequences can be determined by algorithms well known in the art. Preferably, the degree of identity is to he determined by comparing two optimally aligned sequences over a comparison window, where the fragment of amino acid sequence in the comparison window may comprise additions or deletions (e.g gaps or overhangs) as compared to the reference sequence (which does not comprise additions or deletions) for optimal alignment. The percentage is calculated by determining the number og positions at which the amino acid residue occurs in both sequences to yield the number of matched positions by the total number of positions in the window of comparison and multiplying the result by 100 to yield the percentage of sequence identity. Optimal alignment of sequences for comparison may be conducted by the local homology algorithm of Smith and Waterman Add. APU. Math.2:482 (1981), by the homology alignment algorithm of Needleman and Wunsch J. MolBiol 48: 443 (1970) by the search for similarity method of Pearson and Lipman Proc Natl Acad Sci (USA) 85: 2444 (1988), by computerized implementations of these algorithms (GAP, BESIFIT, BLAST, PASTA, and TPASTA in the Wisconsin Genetics Software Package, Genetics Computer Group (GGG), 575 Science Dr. Madison, Wi), or by visual inspection. Given that two sequences have been identifies by comparison, GAP and BESTFIT are preferably employed to determine their optimal alignment and thus, the degree of identity. Preferably, the default values of 5.00 for gap weight and o.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NI-pro BNP as long as the said polypeptides have NT-pro RNP properties. NT-pro BNP properties as referred herein are immunological and(or biological properties. Preferably, the NI-pro BNP variants have immunological properties (e.g. epitope composition) comparable to those of NT-pro BNP. Thus, the variants shall be recognizable by the aforementioned means or ligands used for determination of the amount of the natriurelic peptides. Biological and(or immunological NT-pro BNP properties can be detected by the assay described in Karl et al (Karl 1999, Scand J. Clin Invest 230: 177 ― 181), Yeo et al (Yeo et al Clinical Chimica Acta 338: 107 115).Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label to the peptides.

Determining the amount of GDF 15 or a variant thereof or a natriuretic peptide or a variant thereof or any other peptide or polypeptide referred to in this specification relates to measuring the amount or concentration, preferably semiquantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal - may be obtained e.g. by measuring an intensity value of a specific physical or chemical property of the peptide or polypeptide. Indirect measuring includes measuring of a signal obtained from a secondary component (i.e. component not being the peptide or polypeptide itself) or a biological read out system e.g. measurable cellular response, ligands, labels, or enzymatic reaction products.

In accordance with the present invention, determining the amount or a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be corrected directly or indirectly (e.g. reverse- proportional) to the amount of polypeptide present in the sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers. NMR analyzers or chromatography devices. Further, methods include micro-plate ELISA-based methods, fully automated or robots immunoassays(available for e.g on ELECSYS analysers), CBA (an enzymatic Cobalt Binding Assay, available for example on Roche-Hitachi Analyzers) and latex agglutination assays (available for example on Roche Hitachi analyzers).

Preferably, determining the amount of a peptide or polypeptide comprises the steps(a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g, a peptide, polypeptide or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or an NMR spectrum specific for the peptide or polypeptide.

Determining the amount of a peptide or polypeptide may, preferably, comprises the steps of (a) contacting the peptide with a specific ligand (b) (optionally) removing non-bound ligand (c) measuring the amount of bound ligand. The bound ligand will generate an intensity signal. Binding according to the present invention includes both covalent and non-covalent binding. A ligand according to the present invention can he any compound, e.g. a peptide, polypeptide, nucleic acid, or small molecule, binding to the peptide or polypeptide described herein Preferred ligands include antibodies, nucleic acids, peptides or polypeptides such as receptors or binding partners for the peptide or polypeptide and fragments thereof comprising the binding domains for the peptides, and aptamcrs, e.g. nucleic acid or peptide aptamers. Methods to prepare such ligands arc well known in the art. For example, identification and production of suitable antibodies or aptamers is also offered by commercial suppliers. The person skilled in the art is familiar with methods to develop derivates of such ligands with higher affinity or specificity. For example random mutations can be introduced into the nucleic acids, peptides or polypeptides. These derivates can then be tested for binding according to screening procedures known in the art. e.g. phage display. Antibodies as referred to herein include both polyclonal and monoclonal antibodies as well as fragments thereof, such as Fv, Fab and F(ab)2 fragments that arc capable of binding antigen or hapten. The present invention also includes singlechain antibodies and humanized hybrid antibody wherein amino acid sequences of a nonhuman donor antibody exhibiting a desired antigen-specificity arc combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("crossreact" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, is it can still be distinguished and measured unequivocally, e.g. according to its size on a Western Blot, or by its relatively higher abundances in the sample. Binding of the ligand can be measured by any method known in the art. Preferably, said method is semi-quantitative or quantitative. Suitable methods are described in the following.

First binding of a ligand may be measured directly e.g by NMR or surface plasmon resonance.

Second, if the ligand also serves as it substrate of an enzymatic activity of the peptide or polypeptide of interest an enzymatic reaction product may be measured (e.g. the amount of a protease can be measured by measuring the amount of cleaved substrate, e.g. on a Western blot). Alternatively, the ligand may exhibit enzymatic properties itself and the "ligand/peptide or polypeptide complex on the ligand which was bound by the peptide or polypeptide, respectively, may be contacted with a suitable substrate allowing detection by the generation of an intensity signal. For measurement of enzymatic reaction products, preferably the amount of substrate is saturating The substrate may also he labelled with a detectable label prior to the reaction. Preferably, the sample is contacted wilh the substrate for an adequate period of time. An adequate period of time refers to the time necessary for a detectable, preferentially measurable, amount of product to be produced. Instead of measuring the amount of the product, the time necessary for appearance of a given (e.g. detectable) amount of product can be measured.

third, the ligand may be coupled covalently or non-covalently to a label allowing detection and measurement of the ligand. Labeling may be done by direct or indirect methods. Direct labelling involves coupling of the label directly /covalently or noncovalently) to the ligand. Indirect labelling involves binding (covalently or noncovalently) of a secondary ligand to the first ligand. The secondary ligand should specifically bind to the first ligand. Said secondary ligand may be coupled with a suitable label and/or be the target (receptor) of a tertiary ligand binding to the secondary ligand. The use of secondary, tertiary or even higher order ligands is often used to increase the signal. Suitable secondary and higher order ligands may include antibodies, secondary antibodies, and the wellknown streptavidin-biotin system (Vector Laboratories, Inc). The ligand or substrate may also be "tagged" with one or move tags as known in the art. Such tags may be targets for higher order ligands. Suitably tags include biotin, dioxygenin, His-Tag, Glutathion-S-Transferase, FLAG, GFP, mye-tag, influenza A virus haemagglutinin (HA), maltose binding protein, and the like. In the case of peptide or polypeptide, the tag is preferably at the N-terminus and or C-terminus. Suitable labels are any labels detectable by an appropriate detection method. Typical labels include gold particles, latex beads, acridian ester, luminol, ruthenium, enzymatically active labels, radioactive labels, magnetic labels (e.g. magnetic beads", including paramagnetic and supramagnetic labels), and fluorescent labels. Enzymatically active labels include e.g horseradish peroxidase, alkaline phosphatase, beta-Galactosidase, luciferase and derivates thereof. Suitable substrates for detection include di-amino-benzidine (DAB), 3,3 - 5,5'-tetramethylbenzinine, NBT-BCIP ( 4-nitro blue tetrazolium chloride and 5-bromo-4-chloro-3-indolyl-phosphate, available as ready-made stock solution from Roche Diagnostics). CDP-Star ( Amersham Bioseiences). ECF (Amersham Bioticiences)..A suitable enzyme-substrate combination may result in a colored reaction product, fluorescence of chemoluminescence, which can he measured according to methods known in the art (e.g. using a light-sensitive film or a suitable camera system). As for measuring the enzymatic reaction, the criteria given above apply analogously. Typical fluorescent labels include fluorescent proteins (such as GFP and its derivates). Cy3, Cy5, Texas Red, Fluorescent, and the Alexa dyes (e.g. Alexa 568). Further fluorescent labels are available e.g. from Molecular Probes (Oregon). Also the use of quantum dots as fluorescent labels is contemplated. Typical radioactive labels include 35 S, 1251, 32 P, 33P and the like. A radioactive label can be detected by any method known and appropriate, e.g. a light-sensitive film or a phosphor imager. Suitable measurement methods according to the present invention also include precipitation (particularly immunoprecipitation), electrochemiluminescence (electro generated chemiluminescence), RIA (radiommunoassay), ELISA (enzyme linked immunosorbent assay), sandwhich enzyme immune tests, electrochemoluminecence sandwich immunoassays (ECLIA), dissocialtion-enhanced lanthanide fluoro immuno assay (DELFIA), scintillation proximity assay (SPA), turbidimetry, nephrolometry, latex enhanced turbdimetry or nephrelometry, or solid phase immune tests. Further methods known in the art ( such as gel electrophoresis, 2 D gel electrophoresis, SIDS polyacylamid gel eletrophoresis (SDS PAGE), Western blotting and mass spectrometry), can he used alone or in combination with labelling or other detection methods as described above.

The amount of a peptide or polypeptide may be also preferably, determined as follows (a)contacting a solid support comprising a ligand for the peptide or polypeptide as specified above with a sample comprising the peptide or polypeptide and (b) measuring the amount peptide or polypeptide which is bound to the support. The ligand, preferably chosen from the group of nucleic acids, peptides, polypeptides antibodies and aptamers, is preferably present on a solid phase support in immobilized form. Materials for manufacturing solid support are well known in the art and include, inter alia, commercially available column material, polystyrene beads, latex beads magnetic beads, colloid metal particles, glass and/or silicon chips and surfaces, nitrocellulose strips, membranes, sheets, duracytes, wells and walls of reaction trays, plastic tubes etc The ligand or agent may be bound to many different carriers. Examples of well-known carriers include glass, polystyrene, polyvinyl chloride, polypropylene, polyethylene, polycarbonate dextran, nylon, amyloses, natural and modified celluloses, polyacylamides agaroses, and magnetide. The nature of the carrier can be either soluble or insoluble for the purposes of the invention Suitable method for fixing/immobilizing said ligands are well known and include but are not limited to ionic, hydrophobic, covalent interactions and the like. It is also contemplated to use "suspension arrays" as arrays according to the present invention (Nolan 2002, Trends Biotechnol, 20 (1) 9 - 12), In such suspension arrays, the carrier, e.g. microbead or microsphere, is present in suspension. The array consists of different microbeads or microsheres_{,} possibly labelled, carrying different ligands. Methods of producing such arrays, for example based on solid-phase chemistry and photo-labile protective groups, are generally known (US 5.744,305),

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurement, e.g. intensity values in mass spectra or NMR spectra. Moreover, encompassed are all values or parameters which are obtained hy direct measurements specified elsewhere in this description, e.g. response levels determined from biological read out systems in response to the peptides or intensity signals obtained from specifically bound ligands. It is to be understood that values correlating to the aforementioned amounts or parameters can also he obtained by all standard mathematical methods,

The term "comparing" as used herein encompasses comparing the amount of the peptide or polypetide by the sample to be analysed with an amount of a suitable reference source specified elsewhere in this description. It is to be understood that comparing as used herein refers to a comparison of corresponding parameters or values, e.g. an absolute amount is compared to an absolute reference amount while a concentration is compared to a reference concentration or an intensity signal obtained from a test is compared to the same type of intensity signal of a reference sample. The comparison referred to in step (b) of the method of the present invention may be carried out manually or computer assisted For a computer assisted comparison, the value of the determined amount may be compared to values corresponding to suitable references which are stored in the database by a computer program- The computer program may further evaluate the result of the comparison, i.e. automatically provide the desired assessment in a suitable output format- Based on the comparison of the amount determined instep a) and the reference amount, it is possible to predict the risk of a subject of suffering one or more Complications referred to herein- therefore, the reference amount allows identifying those diabetes mellitus patients which are at risk to suffer complications referred to herein.

Accordingly the term "reference amount" as used herein refers to an amount which allows predicting whether a diabetes mellitus patient is at (increased) risk of suffering from complications specified herein. Accordingly, the reference may either be derived from (i) a diabetes mellitus patient known to have suffered from the specified complication (ii) a diabetes mellitus patients known to have not suffered from the specified complication. Moreover the reference value may define a threshold value whereby an amount lager that the threshold value shall be indicative for a subject at (increased) risk to develop the specified complication while an amount lower than the threshold amount shall be an indicator for a subject not ( or at low ) risk to develop the specified complication. Reference amount in the context of the present invention indicates whether the patient with diabetes is at increased risk of foot ulcer, values below the reference value indicate that the risk is deocased, values above the reference value indicate that the risk is increased. Risk of development of foot ulcer in the context of the present invention is under stood as development of foot ulcer preferably within one month, 2 months, 3 months. 6 months, 1 year, 2 year, 5 years, and more preferably 10 years. 15 years, 20 years and longer. The reference amount for an individual subject may vary depending on various physiological parameters such as age, gender, ethnicity or specific subpopulations as well as on the means used for the determination of the peptide or polypeptide referred to herein. A suitable reference amount may be determined by the method of the present invention from a reference sample to be analysed together, i.e. simultaneously or subsequently, with the test sample. A preferred reference amount serving as a threshold may be derived from the upper limit of normal (UI.N)_{:} i.e. the upper limit of the physiological amount to be found in the population of apparently healthy subject. The ULN for a given population of subjects can be determined by various well known techniques. A suitable technique may be to determine the median of the population for the peptide or the polypeptide amounts to be determined in the method of the present invention.
A referred threshold (i.e. reference amount) for GDI 15 is in the context of the present invention 1000 pg/ml

Thus, the reference amount defining a threshold amount for GDF ) 15 as referred to in accordance with the present invention is 1500 pg/ml, more preferably 1200 pg/ml, most preferably 1000 pg/ml.

A preferred threshold level (i.e. reference amount) for NT-pro BNP in the context of the present invention is 60 pg/ml.

Thus, the reference amount defining a threshold amount for NT-pro BNP as referred to in accordance with the present invention is 150 pg/ml, more preferably 100 pg/ml, most preferably 60 pg/ml

In another preferred embodiment of the present invention, in addition to GDF 15, a natriuretic peptide, more preferably a R type natriuretic peptide and most preferably NT-pro RNP is measured and a ratio between GDF 15 and a natriuretic peptide is formed. Most preferably a ratio of GDF 15 and NT-pro BNP is formed.

A preferred reference ratio formed from the determination of GDF15/NT-pro BNP in the context of the present invention is 10

Thus, the reference amount defining a threshold for GDF15/NT-pro BNP as referred to in accordance with the present invention is 20, preferably 15 and most preferably 10.

In the context of the present invention a value below the reference or threshold level or ratio indicates that the patient with diabetes mellitus is preferably at decreased risk to develop foot ulcer.

In the context of the present invention a value above the reference or threshold level or ratio indicates that the patient with diabetes mellitus is preferably at increased risk to develop fool ulcer

Advantageously, it has been found in the study underlying the present invention that GDF 15 or a ratio formed from GDI 15 and a natriuretic peptide, preferably NT-pro BNP is a reliable diagnostic marker for predicting the risk of a diabetes mellitus patient to suffer from diabetic foot ulcer complication. Thanks to the present invention the risk stratification can easily be performed, allowing to initiale medical, or physical treatment of the patient, including adapting the patients life style. In case the patients risk turns out to be non existent or low, a time and/or cost intensive or, as the case may be dangerous therapy can be avoided. Thus the method of the present invention will be beneficial for the health system in that resources will be saved. It is to be understood that according to the method of the present invention described herein above and below, the amount of GDF 15 or a ratio formed from GDF 15 and a natriuretic peptide, preferably from GDF 15 and NT-pro BNP or means for the determination thereof can be used for the manufacture of a diagnostic composition for identifying a subject being susceptible for a local or systemic intervention.

The term foot ulcer as used herein refers to a skin lesion of the foot which extents to the soft tissue below the skin and which had developed from skin injury- Foot ulcer may be superinfected. Causes of superinfection include fungi and preferably bacteria. Superinfection by bacteria include gram positive and gram negative bacteria. Preferred gram positive bacteria are S. aureus, Staphylococcus species, Enterococcus species. Gram negative bacteria include Pneudomonas aerogenosa, Enterobacteriaceae, Proteus species, and Echerichia coli and Klebsiella species-Resides the aerobic bacteria decribed above, anaerobic superinfections may occur including Bacteroides species. Also. MRSA (Methicillin resistant Straphylococcus aureus) can be found foot ulcer may become chronic and persist for 1 week, 2 weeks, 3 weeks, I month 2 months, 3 months, 6 months and longer preferably for I month , more preferably for 3 months. Infection of the foot ulcer may extend to the bone to cause chronic bone infection termed osteomyelitis.

The present invention, furthermore, relates to a method of assessing the risk of a diabetes mellitus preferably type 2 most preferably type 1 diabetes mellitus patient to suffer from foot ulcer, the method comprising
a) determination of the amount of GDF 15 in sample of a type I diabetes patient; and
b) comparing the amount of GDF 15 determined in step a) to a reference amount, there by assessing the said risk.

the term "assessing the risk" as used herein means estimating the probability whether a subject will in the future suffer from foot ulcer Assessing the risk in the context of the present invention preferably estimates the risk of a diabetes mellitus patient to suffer from foot ulcer in I months, 2 months, 3 months 6 months, I year, 2 years, 5 years and more preferably 10 years. 15 years and 20 year whereas the risk is decreased if GDF 15 is below the threshold level and increased if GDF 15 is above the threshold level. As will be understood by those skilled in the art, such an assessment is usually not intended to be correct for all (i.e. 100 %) of the subjects to be identified (e.g. a cohort in a cohort study). Whether a portion is statistically significant can be determined without further ado by the person skilled in the art using various well known statistic evaluation tools, e.g. determination of confidence intervals, p-value determination. Student's t-test, Mann-Whitney test etc.. Details can be found in Dowdy and Wcardan. Statitics for Research, John Wiley & Sons , New York 1983. Preferred confidence intervals are at least 90 %, at least 95 %, at least 97 %, at least 98 % or at least 99 %. The p-values are preferably 0.1, 0.05, 0.01, 0.005, or 0.0001. More preferably, at least 60 %, at least 70 %, at least 80 % or at least 90 % of the subjects of a population can be property identified by the method of the present invention.

In a preferred embodiment of the method of the present invention, the said reference amount is 1000 pg/ml.

More preferably, an amount of GDF 15 larger than the reference is indicative for an elevated risk of foot ulcer.

In another embodiment of the present invention a ratio between GDF15 and N1 pro RNP is formed, the said reference amount using a GDF15/NI.-pro BNP ratio is 10

More preferably a GDF 15/NT-pro BNP ratio larger than the reference (ratio) is indicative for an elevated risk of foot ulcer.

Similarly, preferably a GDF 15/NT-pro BNP ratio lower than the reference (ratio) is indicative for a decreased risk of foot ulcer

The expression "predicting the risk of suffering from a complication" as used herein means that a subject ( i.e. a diabetes mellitus patient) to be analyzed by the method of the present invention is allocated either into the group of subjects of a population having a normal ,i.e. non elevated, risk for the said complications, or into a group of subjects having a significantly elevated risk. An elevated risk as preferred to in accordance with the present invention means that the risk of complication within a predetermined predictive window is elevated significantly for a subject with respect to the average risk for complication in a population of subjects.

In principle it has been found that GDI 15 or means for determining GDF 15 can be used for the manufacture of a diagnostic composition for predicting whether a patient with type 1 diabetes mellitus is at risk of said complication.

The present invention further relates to a method of deciding on the administration of life style changes or medicaments in a diabetes mellitus patient being susceptible to suffer from a foot ulcer as preferably a consequence of foot injury, the method comprising
a) determining the amount of CDF 15 in a sample of a diabetes mellitus patient
b) comparing the amount of GDF 15 in step a) to a reference amount and
c) deciding on the said administration.

Preferably life style changes in the context of the present invention relate but are not limited to choice of appropriate foot wear, frequent and careful foot care and frequent physician visits, specifically is case of obvious or suspected abnormalities or changes of the skin of the foot.

Preferably, the said therapy to be selected fur the subject by the method of the present invention said therapy is a drug based therapy. More preferably said therapy is a hyperbaric oxygen therapy with and without standard wound care including but not limited to dressing changes and local debridements.

Another preferred therapy is early antifungal and/or antibiotic therapy. Preferred medicaments arc ampicillin, sulbactam, impedenem, cilastin, linezolid, celtriaxone, metronidazole, ticarcillin, clavulanate, ticarcillin, piperacillin, tazobactam, sulbactum, vancomycin, daptomycin, aztrconam, and ertapenem.

Advantageously, by determining the GDF 15 amount in a sample of a subject suffering from foot injury, it can be decided whether a subject will be susceptible for a therapy as referred above. Specifically it is envisaged that a subject having an amount of GDF 15 larger than the reference amount will be suitable to he treated by the aforementioned therapy while a subject with less GDF 15 will not benefit from therapy. Similarly a GDF 15/N1-pro BNP ratio above and below the reference value can be applied

Encompassed by the present invention is, further, a device adopted to carry out the methods of the present invention, comprising means for determining the amount of CDf 15 in a sample of the subject and means for comparing said amount to the reference amount, whereby a diabetes mellitus patient having a predisposition for the complication specified beforehand is identified.

The term "device" as used herein relates to a system of means comprising at least the aforementioned means operatively linked to each other as to allow this prediction. Preferred means for determining the amount of GDF 15, and means for carrying out the comparison are disclosed above in connection with the methods of the invention. How to link the means in an operating manner will depend on the type of means included into the device. For example, where means for automatically determining the amount of the peptides are applied, the data obtained by said automatically operating means can be precessed by, e.g. a computer program in order to obtain the desired results. Preferably, the means are comprised by a single device in such a case. Said device may accordingly include an analyzing unit for processing the resulting data of the evaluation. Alternatively, where means such as test stripes are used for determining the amount of the peptides or polypeptides, the means for comparison may comprise control stripes or tables allocating the determined amount to a reference amount The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with the embodiments relating to the method or the invention above. In such a case, the means are operatively linked.

In that the user of the system brings together the result of the determination of the amount and the diagnostic or prognostic value thereof due to the instructions and interpretations given in the manual. The means may appear as separate devices in such an embodiment and are, preferably, packaged together with the kit. The person skilled in the art will realize how to link the means without further ado. Preferred devices are those which can be applied without the particular knowledge of a specialized clinician e.g. test stripes or electronic devices, which merely require loading with a sample. The results may be given as output of raw data which need interpretation by the clinician. Preferably, the output of the device is however, processed, i.e. evaluate, raw data the interpretation of which does not require a clinician. Further preferred devices comprise the analyzing units/devices (e.g. biosensors, arrays, solid supports coupled to lingands specifically recognizing the peptides, Plasmon surface resonance devices. NMR spectrometers, mass- spectrometers etc) or evaluation units/devices referred to above in accordance with the method of the invention.

Accordingly the present invention also relates to a device for predicting if a diabetes mellitus patient will suffer from diabetic foot complication, comprising means for determining the amount of GDF 15 in a sample of the subject and means for comparing said amount to a reference amount.

Further envisaged is a device for assessing the risk of a diabetes mellitus type 1 patients to suffer from diabetic foot ulcer, comprising means for determining the amount of GDF 15 in a sample of the subject and means for comparing said amount to a reference amount.

The present invention also related to a device for deciding on the administration of medicaments in a type 1 diabetes mellitus patient suffering from diabetic foot ulcer, comprising means for determining the amount of GDF 15 in a sample of the subject and means for comparing said amount to a reference amount.

Furthermore the present invention encompasses a kit adopted to carry out the methods of the present invention, comparing means for determining the amount of GDF 15 in a sample of the subject and means for comparing said amount to a reference amount, whereby a diabetes mellitus patient having a predisposition for the complication as specified beforehand is identified.

The term "kit" as used herein refers to a collection of the aforementioned means, preferably, provided in separately or within a single container, I he container, also preferably, comprises instructions for carrying out the method of the present invention.

The present invention pertains to a kit fur predicting if a diabetes patients will suffer from said diabetic foot ulcer, comprising means for determining the amount of GDF 15 in a sample of the subject and means for comparing said amount to a reference amount.

Also, the present invention relates to a kit for assessing the risk of a diabetes mellitus patient to suffer from diabetic foot ulcer, comprising means for determining the amount of GDF 15 in a sample of the subject and means for comparing said amount to a reference amount.

Finally, the present invention relates to a kit for deciding on the administration of medicaments in a type 1 diabetes mellitus patients being susceptible to suffer from diabetes foot ulcer, comprising means for determining the amount of GDF 15 in a sample of the subject and means for comparing said amount to a reference amount.

All references cited in this specification are herewith incorporated by reference with respect to their entire disclosure content specifically mentioned in this specification.

The following examples shall merely illustrate the invention. They shall not be construed, whatsoever; to limit the scope of the invention.

### Example 1

GDF 15 and N-terminal pro natriuretic peptide CNT-pro BNP were determined with sandwich immuno-assays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubaled with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. The measuring range of the GDF 15 assay is 300 pg to 20000 pg. NT-pro BNP amounts between 2 pg/ml and 35000 pg/ml can be measured.

### Example 2:

A total or 239 patients with stable coronary artery disease which included 186 males and 53 females, mean age 61.2 years were tested, Ischemic heart disease was present in 212 patients as documented by one, two or three vessel disease. Vessel disease was defined as a reduction of the diameter of one or more major vessels by at least 50%. All others had detectable atherosclerosis by angiography but did not meet the criteria of vessel disease. All patients had normal kidney function as defined by creatinine levels within the normal range of the test All patients did not have signs of acute coronary syndrome within 4 weeks prior to the timepoint blood was drawn. Blood was obtained by venipuncture before angiography, was centrifuged within 3U minutes and the resulting scrum was stored art - 20 degrees celsius until tested.

### Example 3:

A total of 891 patients with long standing type 1 diabetes (28,1 year mean age 44,2 year were included into the study. At the timcpoint of study entry all patients were clinically stable without evidence of metabolic decompensation and evidence of acute infection. In addition they had normal kidney function test as assessed by creatinine levels within the normal range, 450 had nephropathy defined as albuminuria between 30 and 300 mg/24 h A history of cardiovascular disease was absent in all patients. Patients were followed for 9.6 years(mean) for the development of diabetic foot complications. Foot ulcer and loss of foot pulse were diagnosed clinically by inspection and palpation respectively. Neuropathy was diagnosed using a 124 Hz vibration fork to assess vibration perception. History of vascular surgery or amputation was recorded using inspection and medical history. Blood was taken by venipuncture at study entry, blood was centrifuged within 30 minutes and stored until analysed.

### Example 4:

In the study groups described in Examples 2 and 3 GDI 15 and NT-pro BNP were tested, the results are outlined in Table 1

**Table 1**

| Percentile | GDF 15 % pg/ml | | N I-pro BNP pg/ml | |
|---|---|---|---|---|
| | CAD | DM | CAD | DM |
| 25% | 515 | 698 | 96 | 29 |
| 50% | 691 | 974 | 266 | 58 |
| 75% | 1045 | 1467 | 928 | 150 |

As can be seen from Table 1 the ratio of GDF 15/NT-pro BNP is significantly different between patients with coronary artery disease (CAP) and evidence of cardiac dysfunction as assessed by NT-pro BNP levels and patients with diabetes mellitus (DM). The ratios (GDF 15/NT-pro BNP) calculated at the different percentiles were 5.3,2,6 and 1,1 in CAD while they were 24, 17 and 10 in DM patients at the 25 %, 50 % and 75 % percentile respectively. In patients with CAD and heart failure GDF 15 has been clearly linked to heart failure and cardiovascular disease, this is however apparently not (or not only) the case in diabetes mellitus.

### Example 5:

Using the GDF 15 median of 974 pg/ml risk factors for development of foot ulcer were assessed in patients with type 1 diabetes mellitus. Sixtysix patients in the low GDF 15 and 59 patients in the high GDF 15 group developed neuropathy (.ns.), indicating that GDF 15 is not involved in the development of neuropathy.

Vascular complications (loss of foot pulse) were seen in 29 patients in the low GDF 15 group and 46 in the high GDF 15 group. This result was marginally statistically different (p = 0.04)

Foot ulcer occurs in 76 patients in the low GDF 15 group and in 107 in the high GDF 15 group (p below 0.001). Among the patients in the high GDF 15 group foot ulcer was present in 69 patients in the highest percentile ( GDF 15 above 1476 pg/ml).

As can be derived from the above analysis, GDF 15 is apparently associated with delayed wound healing and a preferred indicator thereof in patients with diabetes mellitus. This offers the opportunity to identity patients with type 1 diabetes with an increased risk to proceed from injury to ulcer with the consequence of improved surveillance and early intervention.

## Claims

1. A method of predicting the risk of a diabetes mellitus patients to suffer from diabetic foot ulcer complication, the method comprising
a) determining the amount of GDF 15 in a sample of a diabetes mellitus patient and
b) comparing the amount of GDF 15 determined in step a) to a reference amount, thereby predicting the risk

2. A method of claim 1, wherein the amount of GDF 15 larger than the reference amount predicts an increased risk to suffer from diabetic foot complication.

3. A method of claim 1, wherein, wherein the amount of GDF 15 lower that the reference amount predicts a decreased risk to suffer from diabetic foot complication.

4. A method according to claims 1 to 3, wherein the patient suffers from type I diabetes mellitus.

5. The method of claims 1 and 4, wherein said reference amount for GDF 15 is 1000 pg.

6. The method of claims 1 to 3, wherein in addition to GDF 15
a) a natriuretic peptide or a variant thereof is measured and
b) a ratio of GDF 15 and a natriuretic peptide or a variant thereof is formed.

7. The method of claim 6 wherein the natriuretic peptide is a B type natriuretic peptide.

8. The method of claim 6 wherein the B type natriuretic peptide in NT-pro BN P.

9. The method of claim 6 and 8 wherein a GDF 15 NT-pro BNP ratio larger than the reference ratio is indicative for an increased risk from foot ulcer.

10. The methods of Claim 6 and 8 wherein a GDF 15/NT-pro RNP ratio lower than the reference ratio is indicative for a decreased risk of foot ulcer.

11. The method of claims 6 and 8 to 10 wherein the GDF 15/NT-pro BNP reference ratio is 10.

12. A method for assessing the risk of a diabetes mellitus patient preferably type 2 and more preferably type 1 diabetes mellitus patient to suffer from diabetic foot ulcer complication, the method comprising
a) determining the amount of GDF 15 in a sample of a diabetes patient; and
b) comparing the amount of GDF IS determined in step a) to a reference amount, thereby assessing said risk.

13. A method of deciding on the administration of life style changes or application or changes of medicaments in a diabetes patient being susceptible to diabetic foot ulcer, the method comprising
a) determining the amount of GDF 15 in a sample of a type 1 diabetes patient and
b) comparing the amount of GDF 15 determined in step a) to a reference amount and
c) deciding on the said administration.

14. A device for predicting if a diabetes mellitus patient with suffer from diabetic foot ulcer, comprising means for determining the amount of GDF 15 in a sample of the subject and means for comparing said amount to a reference amount.

15. A kit for predicting if a diabetes mellitus patients will suffer from Diabetic foot ulcer complication comprising means for determining the Amount of GDF 15 in a sample of the subject and means for comparing Said amount to reference amount.
